# Europäisches Patentamt

(19) **European Patent Office**

**Office européen des brevets**

(11) Veröffentlichungsnummer: **0 011 126**
**B2**

(12) **NEUE EUROPÄISCHE PATENTSCHRIFT** —

(45) Veröffentlichungstag der neuen Patentschrift:
30.04.86

(21) Anmeldenummer: 79103836.7

(22) Anmeldetag: 08.10.79

(51) Int. Cl.⁴: **C 07 D 301/32**, A 62 C 3/00,
**B 01 J 19/00**

(54) Verfahren zum Schutz Ethylenoxid erzeugender und verarbeitender Anlagen vor einem Ethylenoxid-Zerfall und Anlagen zur Erzeugung und Verarbeitung von Ethylenoxid, die gegen einen Ethylenoxid-Zerfall geschützt sind.

(30) Priorität: 20.11.78 DE 2850254

(43) Veröffentlichungstag der Anmeldung:
28.05.80 Patentblatt 80/11

(45) Bekanntmachung des Hinweises auf die Patenterteilung:
24.02.82 Patentblatt 82/8

(45) Bekanntmachung des Hinweises auf die Entscheidung
über den Einspruch:
30.04.86 Patentblatt 86/18

(84) Benannte Vertragsstaaten:
BE FR GB IT NL SE

(73) Patentinhaber: HÜLS AKTIENGESELLSCHAFT,
Patentabteilung / PB 15 - Postfach 13 20,
D-4370 Marl 1 (DE)

(72) Erfinder: Beestermöller, Winfried, Uckermarkstrasse 4,
D-4370 Marl (DE)
Erfinder: Böhm, Georg, Dr., Im Frett 3, D-4370 Marl (DE)
Erfinder: Erberich, Hans-Jürgen, Dr., Leverkusener
Strasse 12, D-4370 Marl (DE)
Erfinder: Grosse-Wortmann, Hans, Dr., Freiheiter Weg 7,
D-4358 Haltern-Lippramsdorf (DE)
Erfinder: Hinz, Rudolf, Bitterfelder Strasse 12,
D-4370 Marl (DE)
Erfinder: Kutter, Hans-Christian, Dormagener
Strasse 40, D-4370 Marl (DE)
Erfinder: Langheim, Franz,
Karl-Friederich-Gauss-Strasse 19,
D-4350 Recklinghausen (DE)
Erfinder: Rauch, Klaus, Dr., Bitterfelder Strasse 9a,
D-4370 Marl (DE)
Erfinder: Ueberschaer, Horst, Bitterfelder Strasse 2,
D-4370 Marl (DE)
Erfinder: Vangermain, Erwin, Dr., Leverkusener
Strasse 1, D-4370 Marl (DE)

(56) Entgegenhaltungen:
CH - A - 179 586
DE - A - 2 032 071
FR - A - 1 492 999
FR - A - 2 258 884
FR - A - 2 410 540
US - A - 3 557 243

HYDROCARBON PROCESSING, Band 56, September
1977, Houston, US, A.D.Monroy et al.: "Stop fires in EO
plants", Seiten 175,176
CHEMIE-INGENIEUR-TECHNIK, Band 51, Januar 1979,
Weinheim, DE., E.W.SCHOLL et al.: "Vorbeugende und
konstruktive Schutzmassnahmen gegen Gas- und
Staubexplosionen", Seiten 8-14

(56) Entgegenhaltungen: (Fortsetzung)
VFDB-Zeitschrift (Vereinigung zur Förderung des
deutschen Brandschutzes), Jahrgang 1970, S. 31-37
VDI-Wärmeatlas (1977) Kapitel Le 1
Chemie-Ing.-Technik 42 (1970) S. 85-86
S.A. Miller, Ethyiene, ITS Industrial Derivatives (1969) S.
545
Kirk-Othmer, Encyclopedia of Chemical Technology (3.
Aufl. 1980) S. 451

EP 0 011 126 B2

## Beschreibung

Die Erfindung betrifft ein Verfahren zum Schutz von Ethylenoxid erzeugenden und verarbeitenden Anlagen vor einem Ethylenoxid-Zerfall und Anlagen zur Erzeugung und Verarbeitung von Ethylenoxid, die gegen den Zerfall des Ethylenoxid, die gegen den Zerfall des Ethylenoxids geschützt sind.

Die Erfindung verfolgt den Zweck, die Betriebssicherheit von Anlagen zur Erzeugung und Verarbeitung von Ethylenoxid zu erhöhen.

Wegen der Eigenschaften des Ethylenoxids wie Leichtflüchtigkeit, Giftigkeit, Polymerisations- und Zerfallsneigung sind umfangreiche Sicherheitsmaßnahmen beim Umgang mit diesem Stoff zu beachten. Die einschlägigigen gesetzlichen Bestimmungen, die sich auf die Lagerung und den Transport von Ethylenoxid beziehen, tragen dieser Tatsache Rechnung, indem sie die Inertisierung der Dampfphase vorschreiben, z. B. durch eine Stickstoffüberlagerung. Diese Sicherheitsvorkehrungen lassen sich jedoch aus verfahrenstechnischen Gründen nicht ohne weiteres auf Anlagen zur Herstellung und Weiterverarbeitung von Ethylenoxid übertragen.

Der vorliegenden Erfindung liegt die Aufgabe zugrunde, für Anlagen zur Erzeugung und Verarbeitung von Ethylenoxid Schutzvorrichtungen vorzusehen, die den Verfahrensablauf nicht beeinträchtigen und die Anlagen vor einem Zerfall des Ethylenoxids schützen.

Diese Aufgabe wird erfindungsgemäß gelöst durch das Abschotten einzelner oder mehrerer Apparate der Anlage durch den Einbau von an sich bekannten Zerfallssperren, die druckstoßfeste Auslegung aller Apparate und Rohrleitungen, für die der maximale Druck, der sich bei einem Zerfall des Ethylenoxids ergibt, mit hinreichend großer Zuverlässigkeit vorausberechnet werden kann, die schnelle Inertisierung des Inhalts einzelner oder mehrerer Apparate, die nicht dauernd im inertisierten Zustand betrieben werden können im Falle einer Störung.

Durch das Abschotten einzelner oder mehrerer Apparate wird die Ausbreitung eines Ethylenoxid-Zerfalls verzögert. Als Zerfallssperren sind Füllkörperschüttungen geeignet, wie sie vom Umgang mit Acetylen her an sich bekannt sind. Auch können einzelne Apparate (z. B. die Destillationsvorlage) durch entsprechende Einbauten selbst als Zerfallssperre wirken. Zerfallssperren mit Füllkörperschüttungen sollen senkrecht durchströmt werden, um größere freie Kanäle in Strömungsrichtung zu vermeiden.

Zerfallssperren obengenannter Konstruktion sind in jedem Fall in der Lage, einen einlaufenden Ethylenoxid-Zerfall zu verzögern. Sind jedoch Betriebszustände (höhere Betriebsdrücke) möglich, bei denen sich eine stationäre Zersetzungsflamme vor der Zerfallssperre ausbilden kann, dann ist die Standzeit der Sperre wegen der zunehmenden Erwärmung begrenzt. In diesen Fällen muß daher der Raum hinter der Sperre, also der Inhalt des zu schützenden Apparates, inertisiert sein oder in der zur Verfügung stehenden Zeit inertisiert werden.

Die Standzeit der Zerfallssperre läßt sich durch geeignete Maßnahmen verlängen, z. B. durch eine innere Berieselung mit Wasser, wobei die Berieselungsdichte hinreichend groß zu bemessen ist, damit eine unzulässige Erwärmung der Füllkörper mit Sicherheit vermieden wird.

Sämtliche Apparate und Rohrleitungen, für die der maximale Druck, der sich bei einem Zerfall des Ethylenoxids ergibt, mit hinreichend großer Zuverlässigkeit vorausberechnet werden kann, werden druckstoßfest ausgelegt. Dabei versteht man unter druckstoßfester Auslegung, daß ein Bauteil für den Fall eines Druckstoßes kurzzeitig bis an die Streckgrenze des Baumaterials belastet werden darf. Diese Auslegungsform ist sinnvoll, da hierdurch selbst bei Auftreten eines Ethylenoxid-Zerfalls eine Zerstörung des Bauteils und damit die mögliche Entspannung der Anlage vermieden wird.

Die druckstoßfeste Auslegung der Apparate und Rohrleitungen ist dann sinnvoll, wenn der Druckanstieg bei einem Ethylenoxid-Zerfall begrenzt ist. Durch Versuche wurde gefunden, daß beim Zerfall von Ethylenoxid-Dampf in Abwesenheit der flüssigen Phase der Enddruck das ca. 10 fache des Anfangswertes nicht übersteigt. Handelt es sich jedoch um Apparate, in denen neben der dampfförmigen auch größere Mengen der flüssigen Phase vorliegen, z. B. die Destillationskolonne, dann läßt sich ein maximaler Enddruck nicht mehr angeben, da der flüssige Vorrat z. T. mitreagiert. Gerade bei diesen Apparaten kommt der raschen Inertisierung eine entscheidende Bedeutung zu.

Größere Anlagenteile, die nicht ständig im inertisierten Zustand gehalten werden können, wie z. B. die Destillationskolonne, werden beim Auftreten kritischer Betriebszustände rasch inertisiert. Dieser Maßnahme ist notwendig, damit über kritisch aufgeheizte Behälterewände, Rohrwände und Zerfallssperren kein Ethylenoxid-Zerfall eingeleitet werden kann.

Die Inertisierung einzelner oder mehrerer Apparate wird durch die gezielte Einspeisung eines geeigneten Gases, vorzugsweise Stickstoff, erreicht. Bei dem System «Kolonne-Kondensator» kann die Einspeisung gleichzeitig an mehreren Stellen erfolgen, wobei die einzelnen Einspeisestellen noch hinsichtlich Öffnungszeitpunkt und Einspeisemenge sinnvoll aufeinander abgestimmt werden müssen. Besonders einfach wird die Inertisierung, wenn man nur eine Einspeisung oberhalb des Sumpfes wählt. Hierdurch werden — bei richtiger Dosierung des Stickstoffs — die Kolonne, der Kondensator und das Dämpferohr inertisiert und die Bildung von Bereichen mit einer kritischen Ethylenoxid-Konzentration wird vermieden. Das gesamte System ist dann inertisiert, wenn an keiner Stelle die Ethylenoxid-Dampfkonzentration den Wert von ca. 50 Vol.-% übersteigt. Der zur Inertisierung benötigte Stickstoff wird zweckmäßigerweise in einem separaten Behälter unter Druck bevorratet.

Die Inertisierung der Destillationskolonne kann auch durch Benutzen des aufsteigenden Dampfes einer geeigneten Component der zu destillierenden Flüssigkeit erfolgen, z. B. die Destillationskolonne

3 **0 011 126** 4

zur Trennung eines Gemisches aus Wasser und Ethylenoxid durch den aufsteigenden Wasserdampf nach dem Schließen der Ventile in der Zulauf- und der Rücklaufleitung inertisiert werden.

Zur Inertisierung sind an Stelle von Stickstoff auch andere Gase, wie z. B. Kohlendioxid, Edelgase, gesättigte gassförmige Kohlenwasserstoffe oder Olefine geeignet.

Da die Inertisierung als Schutzmaßnahme erst nach dem Erkennen eines kritischen Betriebszustandes eingeleitet werden kann, kommt dessen Erfassung und Überwachung durch geeignete Prozeßparameter eine entscheidende Bedeutung zu. Als Überwachungskriterien haben sich unter anderem folgende Größen als geeignet erwiesen: die Temperatur in den Zerfallssperren, der Druck in den großvolumigen Apparaten und die Konzentration des Ethylenoxids in der Umgebungsluft von Anlageteilen, bei denen erfahrungsgemäß Undichtigkeiten auftreten können.

Zerfallssperren wurden bisher nur in Acetylenanlagen eingesetzt, also bei einem zum Zerfall neigenden Gas, dessen Temperatur unter den Betriebsbedingungen der Anlage hinreichend weit oberhalb seiner Kondensationstemperatur liegt. Bisher wurden Zerfallssperren nur bei Abwesenheit der flüssigen Phase des zerfallsfähigen Stoffes als wirksam angesehen.

Bei der Destillation von Ethylenoxid beispielsweise befinden sich jedoch die gasförmige und flüssige Phase des Ethylenoxids im thermodynamischen Gleichgewicht; die Füllkörper in den Zerfallssperren sind in der Regel mit der siedenden flüssigen Phase benetzt.

Wie umfangreiche experimentelle Untersuchungen jedoch zeigten, haben auch Zerfallssperren üblicher Bauart, die mit der flüssigen Phase des zerfallsfähigen Stoffes benetzt sind, eine ausgezeichnete Sperrwirkung. Die Versuche wurden vorzugsweise mit Schüttungen aus Pallringen mit 15 mm Durchmesser aus V4A-Edelstahl durchgeführt; diese Schüttungen waren etwa 1,2 m lang bei einem Durchmesser von etwa 9 cm. Auch Zerfallssperren mit anderen Einbauten, die eine ähnlich große spezifische Oberfläche haben, zeigen eine ähnlich gute Wirkung. Dieses überraschende Ergebnis überwindet also das bisherige Vorurteil gegen die Verwendung von Zerfallssperren bei zum Zerfall neigenden Dämpfen, bei denen also die Füllkörperschüttungen von der flüssigen Phase benetzt sind.

Die Abmessungen der Zerfallssperren, die für den Schutz von Ethylenoxid erzeugenden und verarbeitenden Anlagen geeignet sind, lassen sich folgendermaßen ermitteln:

Die Höhe der Zerfallssperren hängt ab von dem Zeitbedarf $t_i$, der für die Inertisierung der großvolumigen Behälter und Apparate, die von den Zerfallssperren jeweils abgeschottet werden, im Störungsfall notwendig ist, sowie von der Standzeit $t_z$ der Zerfallssperren. Letztere wird bestimmt von der spezifischen Oberfläche der Schüttung und den Zustandsbedingungen im Störungsfall. Um die Ausbreitung des Zerfalls in die durch die Zerfallssperren jeweils abgeschotteten großvolumigen Behälter zu verhindern muß $t_z > t_i$ sein.

Die Standzeit der zerfallssperren ist die Zeit, die die Flammenfront zum Durchdringen der Schüttung benötigt; sie ist proportional zur Höhe H der Zerfallssperre und umgekehrt proportional zur mittleren Ausbreitungsgeschwindigkeit $v_z$ der Flammenfront in der Schüttung der Zerfallssperre:

$$t_z = \frac{H}{v_z}$$

Damit wird

$$\frac{t_z}{t_i} = \frac{H}{v_z \cdot t_i} = S$$

und

$$H = S \cdot v_z \cdot t_i$$

Der Sicherheitsfaktor S muß größer als 1 sein; man setzt $S \geqslant 1,2$, vorzugsweise $S = 1,5$.

Im Zerfallssperren, die mit Pallringen von 15 mm Durchmesser aus V4A-Edelstahl gefüllt sind, beträgt die mittlere Ausbreitungsgeschwindigkeit $v_z$ der Flammenfront von zerfallendem Ethylenoxid $v_z$ = 1 bis 4 cm/s ohne innere Wasserkühlung der Schüttung bei einem Druck oberhalb 15 bar in der Anlage. Unterhalb 15 bar breitet sich die Flammenfront nicht mehr durch die Zerfallssperre aus. Diese Ausbreitungsgeschwindigkeit läßt sich durch innere Kühlung der Schüttung weiter herabsetzen.

Eine Schüttung aus Pallringen 15 mm × 15 mm × 0,4 mm aus V4A-Edelstahl, die durch Rütteln auf ein Schüttgewicht von 500 bis 530 kg/m³ verdichtet ist, hat eine spezifische Oberfläche von 350 bis 370 m²/m³. Werden in Ethylenoxid-Anlagen Zerfallssperren mit anderen Einbauten verwendet, muß deren spezifische Oberfläche in diesem Bereich oder darüber liegen.

Der Querschnitt der Zerfallssperren wird bestimmt durch den Widerstandsbeiwert der Schüttung und den bei Normaldruck der Anlage zulässigen Druckabfall des durch die Zerfallssperre strömenden Gases oder Dampfes:

$$\triangle_p = \zeta \cdot \frac{H}{d_p} \cdot \frac{p}{2} \left(\frac{\dot{V}}{F}\right)^2$$

Dabei ist:

$\triangle_p$ der zulässige Druckabfall an der Zerfallssperre bei Normalbetrieb der Anlaga
$\zeta$ der Widerstandsbeiwert der Schüttung
H die Höhe der Zerfallssperre
$d_p$ eine «charakteristische Abmessung» der Füllkörper
p die Dichte des Gases unter Betriebsbedingungen
$\dot{V}$ der mittlere Volumenstrom des Gases durch die Zerfallssperre
F der Querschnitt der Zerfallssperre

Für eine Schüttung aus Pallringen 15 mm × 15 mm × 0,4 mm aus V4A-Edelstahl ist:

$$\zeta = 3,5 \text{ bis } 5,5$$
$$d_p = 1,5 \text{ cm}$$

Für den Querschnitt der Zerfallssperre fogt daraus

$$F = \dot{V}\sqrt{\frac{p}{2\Delta_p} \cdot \frac{\zeta \cdot H}{d_p}}$$

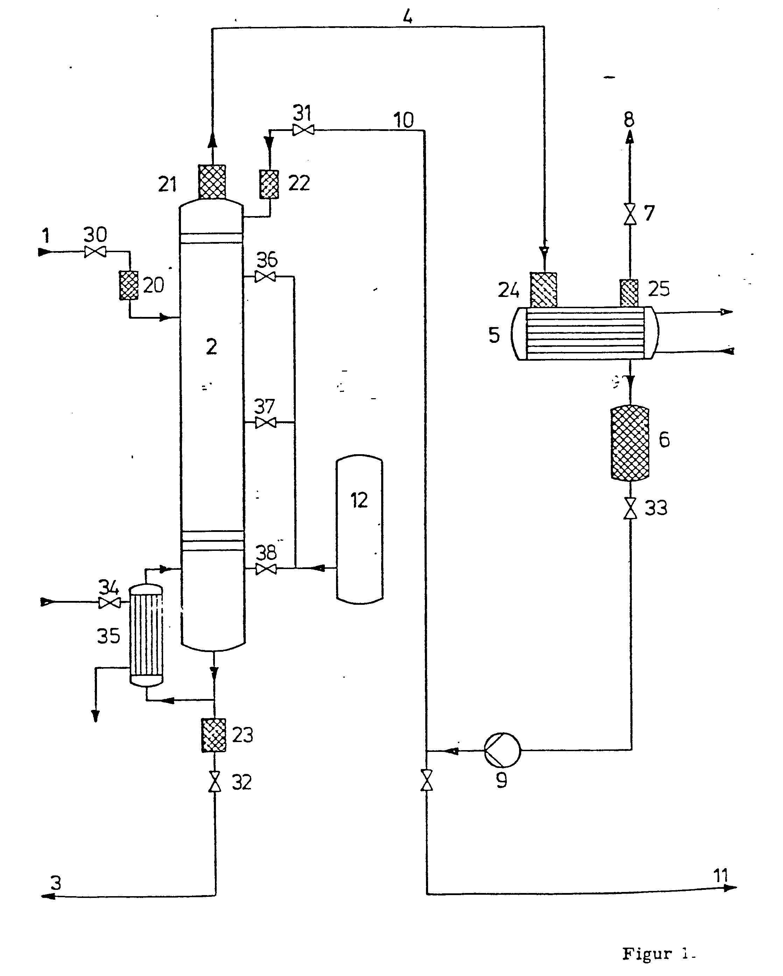

Die Figur 1 verdeutlicht am Beispiel einer Destillationsanlage für ein Flüssigkeitsgemisch mit Ethylenoxid als der leichtersiedenden Komponente den Einsatz der Zerfallssperren und ein Konzept zur Inertisierung. Über die Zulaufleitung (1) wird die zu destillierende Ethylenoxid-haltige Flüssigkeit der Destillationskolonne (2) zugeführt. Die schwerersiedende Komponente wird als Sumpfprodukt über die Rohrleitung (3) abgezogen. Der aufsteigende Ethylenoxid-Dampf gelangt über die Rophrleitung (4) zum Konsdensator (5), wird hier kondensiert und in der Vorlage (6) gesammelt. Die nicht kondensierten Komponenten werden über das Druckhalteventil (7) in der Leitung (8) ausgeschleust. Aus der Vorlage wird das flüssige Ethylenoxid mittels der Pumpe (9) über die Leitung (10) als Rücklauf am Kopf der Kolonne wieder aufgegeben und über die Leitung (11) der weiteren Verarbeitung zugeführt. Der zur Inertisierung erforderliche Stickstoff wird in dem Druckbehälter (12) bevorratet.

Durch den Einbau der Zerfallssperren (20) bis (23) in die Zulauf-, Dämpfe-, Rücklauf- und Sumpfproduktleitung wird die Destillationskolonne vor einem einlaufenden Ethylenoxid-Zerfall geschützt. Gleiches gilt für den Kondensator, der durch die Zerfallssperren (24) und (25) in der Dämpfe- und Inertgasleitung sowie durch die Ausbildung der Vorlage (6) als Zerfallssperre gesichert wird.

Stellt sich nun ein kritischer Betriebszustand ein, dann werden die Ventile (30), (32), (32), (33) und (7) geschlossen, die Dampfzufuhr zum Umlaufverdampfer (35) wird mittels des Ventils (34) — gegebenenfalls verzögert — unterbrochen, und durch Öffnen der Ventile (36) bis (38) wird der Stickstoff in die Kolonne entspannt, wodurch die Kolonne und der Kondensator inertisiert werden.

## Ansprüche

1. Verfahren zum Schutz von Ethylenoxid erzeugenden und verarbeitenden Anlagen vor einem Ethylenoxid-Zerfall, gekennzeichnet durch das Abschotten einzelner oder mehrerer Apparate der Anlage durch den Einbau von an sich bekannten Zerfallssperren und das druckstoßfeste Auslegen aller Apparate und Rohrleitungen, für die der maximale Druck, der sich bei einem Zerfall des Ethylenoxids ergibt, mit hinreichend großer Zuverlässigkeit vorausberechnet werden kann, und das schnelle Inertisieren des Inhalts einzelner oder mehrerer Apparate, die nicht dauernd im inertisierten Zustand gehalten werden können, im Falle einer Störung.

2. Verfahren nach Anspruch 1, gekennzeichnet durch inneres Kühlen der Zerfallssperren im Falle einer Störung.

3. Verfahren nach Anspruch 1, gekennzeichnet durch Benutzen des aufsteigenden Dampfes einer geeigneten Komponente der zu destillierenden Flüssigkeit zur Inertisierung der Destillationskolonne.

4. Vorrichtung ausschließlich zur Erzeugung und Verarbeitung von Ethylenoxid, gekennzeichnet durch Zerfallsperren, mit denen einzelne oder mehrere Apparate der Anlage, die nicht dauernd im inertisierten Zustand gehalten werden können und die nicht druckstodßfest ausgelegt sind, abgeschottet sind, und druckstoßfest ausgelegte Apparate und Rohrleitungen, für die der maximale Druck, der sich bei einem Zerfall des Ethylenoxids ergibt, mit hinreichend großer Zuverlässigkeit vorausberechnet werden kann, und Druckbehälter zur Bevorratung eines geeigneten Gases für die Inertisierung, die über Ventile enthaltende Rohrleitungen mit den nicht dauernd im inertisierten Zustand gehaltenen Apparaten der Anlage verbunden sind, und Vorrichtungen zum Erkennen eines kritischen Betriebszustandes.

5. Vorrichtung nach Anspruch 4, gekennzeichnet durch die Auslegung einzelner Apparate selbst als Zerfallssperren.

6. Vorrichtung nach Anspruch 4, gekennzeichnet durch Zerfallssperren mit der Höhe

$$H = S \cdot v_z \cdot t_i$$

und dem Querschnitt

$$F = \dot{V}\sqrt{\frac{p}{2\Delta_p} \cdot \frac{\zeta \cdot H}{d_p}}$$

mit
$S$ = Sicherheitsfaktor $\geqq 1,2$
$v_z$ = mittlere Ausbreitungsgeschwindigkeit der Flammenfront in der Zerfallssperre; für zerfallenes Ethylenoxid ist $v_z$ = 1 bis 4 cm/s ohne innere Wasserkühlung bei einem Betriebsdruck oberhalb 15 bar
$t_i$ = Zeitbedarf für die Inertisierung der großvolumigen Behälter
$\dot{V}$ = mittlerer Volumenstrom des Gases
$p$ = Dichte des Gases unter Betriebsbedingungen
$\Delta_p$ = zulässiger Druckabfall an der Zerfallssperre bei Normalbetrieb
$\zeta$ = Widerstandsbeiwert der Schüttung
$d_p$ = charakteristische Abmessung der Füllkörper

## Claims

1. Process for protecting installations, which produce and process ethylene oxide, against decomposition of ethylene oxide, characterised in that individual apparatuses, or a plurality of apparatuses, of the installation are screened off by incorporation of decomposition barriers which are known per se, and all apparatuses and pipelines are designed to withstand pressure shock, for which the maximum pressure which would result on decomposition of the ethylene oxide can be calculated beforehand with sufficiently high reliability, and the contents of individual apparatuses, or of a plurality of apparatuses, which cannot constantly be kept in a state where they have been rendered inert, are rapidly rendered inert in the event of a fault.

2. Process according to Claim 1, characterised in that the decomposition barriers are cooled internally in the event of a fault.

3. Process according to Claim 1, charactrerised in that the rising vapour of a suitable component of the liquid to be distilled is utilised to render the distillation column inert.

4. Equipment exclusively for producing and processing ethylene oxide, characterized by decomposition barriers with which individual apparatuses, or a plurality of apparatuses, of the installation, which cannot constantly be kept in a state where they have been rendered inert and which are not designed to withstand pressure shock, are screened off and apparatuses and pipelines which are designed to withstand pressure shock and for which the maximum pressure which results in the event of decomposition of the ethylene oxide can be calculated beforehand with sufficiently high reliability and pressure vessels for storage of a stuitable gas for rendering the apparatuses contents inert, which are connected by ducts and valves with those apparatuses of the installation which cannot constantly be kept in a state where they have been rendered inert and equipment for detecting critical operating conditions.

5. Equipment according to Claim 4, characterised in that individual apparatuses are themselves designed as decomposition barriers.

6. Equipment according to Claim 4, characterised by decomposition barriers of height

$$H = S \cdot v_z \cdot t_i$$

and cross-section

$$F = \dot{V} \sqrt{\frac{p}{2\triangle_p} \cdot \frac{\zeta \cdot H}{d_p}}$$

where
 $S$ = safety factor $\geqq 1.2$
 $v_z$ = average propagation velocity of the flame front in the decomposition barrier; for decomposing ethylene oxide, $v_z = 1$ to $4$ cm/s without internal water cooling, under en operating pressure of above 15 bar,
 $t_i$ = time required for rendering the contents of the large-volume containers inert,
 $\dot{V}$ = average volumetric flow rate of the gas,
 $p$ = density of the gas under operating conditions,
 $\triangle_p$ = permissible pressure drop at the decomposition barrier in normal operation,
 $\zeta$ = resistance coefficient of the loosely packed material and
 $d_p$ = characteristic dimension ("hydraulic diameter") of the packing bodies.

## Revendications

1. Procédé de protection d'installations fabriqunt et transformant de l'oxyde d'éthylène contre la décomposition de l'oxyde d'éthylène, caractérisé par le fait que l'on isole des appareils individuellement ou plusieurs appareils de l'installation en montant des barrières antidécomposition en elles-même connues, que l'on donne une conception résistantà l'impulsion de la pression à tous les appareils et tuyauteries pour lesquels on peut calculer à l'avance, avec une fiabilité suffisamment grande, la pression maximale qui se produit en cas de décomposition de l'oxyde d'éthylène, et que l'on rend rapidement inerte, en cas de perturbation, le contenu d'appareils individuellement ou de plusieurs appareils qui ne peuvent pas être maintenus en permanence à l'état rendu inerte.

2. Procédé selon la revendication 1, caractérisé par le fait que l'on refroidit intérieurement les barrières anti-décomposition en cas de perturbation.

3. Procédé selon la revendication 1, caractérisé par le fait que l'on utilise la vapeur ascendante d'un constituant approprié du liquide à destiller pour rendre inerte la colonne à distiller.

4. Dispositif exclusivement pour la fabrication et la transformation d'oxyde d'éthylène, caractérisé par des barrières anti-décomposition au moyen desquelles on isole des appareils individuellement ou plusieurs appareils de l'installation qui ne peuvent pas être maintenus en permanence à l'état rendu inerte et qui ne sont pas conçus de manière a résister à l'impulsion de la pression, par des appareils et tuyauteries conçus de manière à résister à l'impulsion de la pression, pour lesquelles on peut calculer à l'avance, avec une fiabilité suffisamment grande, la pression maximale qui se produit en cas de décomposition de l'oxyde d'éthylène, par des récipients à pression dans lesquels on stocke un gaz approprié au traitement qui rend inerte, les récipients étant connectés par des tuyauteries et des valves avec les appareils de l'installation qui ne peuvent pas être maintenus en permanence à l'état rendu inerte et par des dispositifs pour déceler un état de fonctionnement critique.

5. Dispositif selon la revendication 4, caractérisé par le fait que des appareils individuels sont eux-mêmes conçus comme barrières anti-décomposition.

6. Dispositif selon la revendication 4, caractérisé par des barrières anti-décomposition dont la hauteur est

$$H = S \cdot v_z \cdot t_i$$

et dont la section est

$$F = \dot{V} \sqrt{\frac{p}{2\triangle_p} \cdot \frac{\zeta \cdot H}{d_p}}$$

avec

S = coefficient de sécurité supérieur ou égal à 1,2

$v_z$ = vitesse moyenne de propagation du front de flammes dans la barrière anti-décomposition; pour l'oxyde d'éthylène décomposé, $v_z$ = 1 à 4 cm/s sans refroidissement intérieur à l'eau, à une pression de service supérieure a 15 bar,

$t_i$ = temps nécessaire pour rendre inertes les récipients de grand volume,

$\dot{V}$ = débit volumétrique moyen du gaz,

p = densité du gaz dans les conditions de service,

$\Delta_p$ = perte de pression admissible à l'endroit de la barrière anti-décomposition en service normal,

$\zeta$ = coefficient de drésistance du remplissage,

$d_p$ = dimension caractéristique («diamètre hydraulique») des corps de remplissage.

**0 011 126**

Figur 1.